Europäisches Patentamt

European Patent Office

Office européen des brevets

(11) Publication number: **0 501 293 A1**

# EUROPEAN PATENT APPLICATION

(21) Application number: 92102730.6

(51) Int. Cl.5: **A61K 31/70**

(22) Date of filing: **19.02.92**

Pririty: IT 25.02.91 MI91A000474.

(30) Priority: **25.02.91 IT 47491**

(43) Date of publication of application:
**02.09.92 Bulletin 92/36**

(84) Designated Contracting States:
**AT BE CH DE DK ES FR GB GR IT LI LU MC
NL PT SE**

(71) Applicant: **BIORESEARCH S.p.A.
Via Fosse Ardeatine, 2
I-20060 Liscate (Prov. of Milan)(IT)**

(72) Inventor: **Bortolini, Michele
Ludwigstrasse 9/A
W-6720 Speyer(DE)**

(74) Representative: **Gervasi, Gemma et al
NOTARBARTOLO & GERVASI Srl Viale
Bianca Maria 33
I-20122 Milan(IT)**

(54) **Use of S-adenosyl-L-methionine in the treatment of intrahepatic cholestasis determined by total parenteral nutrition.**

(57) The invention relates to the use of S-adenosyl-L-methionine (SAMe) in the form of pharmaceutically acceptable salts in the preparation of injectable pharmaceutical compositions for the prevention and symptomatic treatment of intrahepatic cholestasis determined by total parenteral nutrition.

EP 0 501 293 A1

Prior art

Total parenteral nutrition (TPN) is an important therapeutic aid in the treatment of hyponutritional states consequent on malnutrition or hypercatabolism as observed in chronic inflammatory diseases of the intestine, in hepatic or renal insufficiency, in pancreatite, in coma, in severe trauma, in extended burns and in neoplastic patients. In these cases, TPN is able to improve the quality of life and aid patient survival.

However a patient receiving TPN shows complications independent of the basic pathology but consequent on the TPN itself. The complications observed with greatest frequency are hepatic complications. In particular, 30-60% of patients undergoing TPN develop intrahepatic cholestasis after the first 2-3 weeks of therapy (Fovin-Fortunet et al. Gastroenterology 1982, 82:932-937; Allardyce et al. Surg. Gynecol. Obstet. 1982, 154:641-647; Wagner et al. Am. J. Gastroenterol. 1983, 78:199-202).

Intrahepatic cholestasis is caused by a bile formation defect consequent on damage suffered by the hepatic cell. The pathogenetic factors most frequently claimed are toxicity of certain intravenously administered nutrients, reduced choleresis due to the suspension of oral nutrition, altered carbohydrate/fat equilibrium, endotoxinemia and accumulation of cholestatic substances due to modifications in the intestinal bacterial flora. From the biochemical viewpoint, intrahepatic cholestasis is characterised by the accumulation in the blood of substances normally excreted with the bile, mainly bilirubin and enzymes such as alkaline phosphates and $\gamma$-glutamyltranspeptidase.

An increase in transaminase plasmatic levels (AST, ALT) is also observed, this being an expression of hepatic cytolysis.

Although from a clinical viewpoint intrahepatic cholestasis consequent on TPN may be asymptomatic, it represents an important cause of hepatic lesions which can lead to irreversible damage, such as that observed in cirrhosis. The lack of a current specific pharmacological treatment for this complication makes it necessary to suspend parenteral nutrition therapy whenever biochemical cholestasis alterations are observed, thus denying adequate treatment of the basic pathology.

Summary

We have now found that intrahepatic cholestasis consequent on total parenteral nutrition (TPN) can be prevented or cured by administering S-adenosyl-L-methionine (SAMe) in the form of its pharmaceutically acceptable salts.

The present invention therefore relates to the use of SAMe in the form of pharmaceutically acceptable salts in the preparation of injectable pharmaceutical compositions for the prevention and symptomatic treatment of intrahepatic cholestasis determined by total parenteral nutrition.

This treatment is highly effective, with the absence of side effects.

Detailed description of the invention

The characteristics and advantages of the use of SAMe and its pharmaceutically acceptable salts in the preparation of pharmaceutical compositions for the prevention and symptomatic treatment of intrahepatic cholestasis determined by total parenteral nutrition (TPN) will be more apparent from the following detailed description, which relates to a clinical trial conducted on patients under TPN.

The trial was conducted as a single blind test against an untreated control group, and involved evaluating the effects of S-adenosylmethionine (SAMe) administration on the biochemical parameters of cholestasis and hepatic cytolysis (total and conjugated bilirubin n.v. < 18 $\mu$mol/L and < 4 $\mu$mol/L respectively; alkaline phosphatase n.v. < 2 $\mu$kat/L; $\gamma$-GT n.v. < 0.5 $\mu$kat/L; AST and ALT transaminase n.v. < 0.58 $\mu$kat/L).

37 hospitalised patients of both sexes and of age exceeding 18 years were enrolled for the trial.

Hospitalisation had been necessary for these patients because of the appearance of symptoms requiring diagnosis or because of aggravation of symptoms relating to an already known pathology. Suspension of oral feeding had been prescribed in all these cases, the patients therefore being subjected to total parenteral nutrition (50% glucosate 500 ml; 10% Intralipid 500 ml; Freamine II 500 ml).

Before commencing the parenteral nutrition treatment the patients were randomly assigned to two groups:

Group A: SAMe-1,4-butanedisulphonate was administered to 16 patients intravenously at a daily dose of 800 mg of active substance in association with the parenteral nutrition preparation;

Group B: the parenteral nutrition treatment was administered to 21 patients initially without SAMe. SAMe was associated subsequently, when cholestasis appeared.

The demographic and clinical characteristics of the trial patients are given in Table I.

Seven patients with chronic intestinal inflammatory disease (Chron's disease) received simultaneous treatment with antiinflammatories (1 patient with sulfalazine, 2 patients with corticosteroids and 4 patients with 5-ASA).

All the patients concerned showed normal cholestasis and hepatic cytolysis parameters at the commencement of the trial (Tables I, II, III, IV).

At the commencement of the trial, no statistically significant difference was noted in the 2 treatment groups with regard to demographic and clinical characteristics (Table I).

TABLE I - Demographic and clinical characteristics of the cases studied

| | Group A (16 patients) | Group B (21 patients) |
|---|---|---|
| Age (average, range) | 40 (20-88) | 39 (19-73) |
| Sex (M/F) | 10/6 | 11/10 |
| Diagnosis: | | |
| Chron's disease | 5 | 8 |
| ulcerous colitis | 6 | 6 |
| others | 5 | 7 |
| Concurrent therapy: | | |
| sulfalazine | 0 | 1 |
| corticosteroids | 1 | 1 |
| 5-ASA | 2 | 2 |
| Biochemical parameters: | | |
| total bilirubin ($\mu$mol/L) | 12 ± 1.0 | 10.7 ± 1.0 |
| conjugated bilirubin ($\mu$mol/L) | 2.7 ± 0.5 | 2.9 ± 0.3 |
| alkaline phosphatase ($\mu$kat/L) | 1.4 ± 0.06 | 1.5 ± 0.05 |
| GGT ($\mu$kat/L) | 0.5 ± 0.03 | 0.5 ± 0.03 |
| AST ($\mu$kat/L) | 0.2 ± 0.03 | 0.2 ± 0.02 |
| ALT ($\mu$kat/L) | 0.3 ± 0.02 | 0.3 ± 0.02 |

The biochemical cholestasis and hepatic cytolysis parameters were evaluated every 3 days during the entire trial period (average 28.5 days).

In group A, to which TPN and SAMe were administered, one patient in 16 (6%) (patient No.15, Table II) showed a transient increase in total and conjugated bilirubin on the 20th day of therapy (22.2 $\mu$mol/L and 11.9 $\mu$mol/L respectively), whereas the alkaline phosphatase, $\gamma$-GT, AST and ALT values remained within the normality range (1.7 $\mu$kat/L, 0.4 $\mu$kat/L, 0.2 $\mu$kat/L, 0.4 $\mu$kat/L respectively).

6 days after the appearance of cholestasis, during which the treatment scheme was maintained constant, the total and conjugated bilirubin values became normal and remained normal until the end of treatment (29th day) (Table II). None of the other patients of group A developed intrahepatic cholestasis during the trial period (average 25 days) (Table II).

TABLE II - Group A: Biochemical parameter values at beginning and end of trial (average 25 days) on 16 patients subjected to total parenteral nutrition (TPN) in association with SAMe-1,4-butanedisulphonate (800 mg/day i.v.)

| Pat. | | | Beginning of trial | | | | | | End of trial | | | | |
|------|-----|-----|-----|-----|-----|-----|-----|------|-----|-----|-----|-----|-----|
| No. | TB | CB | AP | GGT | AST | ALT | Day | TB | CB | AP | GGT | AST | ALT |
| 1 | 6.8 | 1.7 | 1.4 | 0.7 | 0.4 | 0.5 | 23 | 5.1 | 1.7 | 1.3 | 0.5 | 0.3 | 0.4 |
| 2 | 12.0 | 3.4 | 1.6 | 0.6 | 0.1 | 0.3 | 29 | 10.3 | 3.4 | 1.7 | 0.6 | 0.2 | 0.4 |
| 3 | 10.3 | 4.1 | 1.3 | 0.4 | 0.3 | 0.4 | 18 | 8.6 | 1.7 | 1.4 | 0.5 | 0.3 | 0.4 |
| 4 | 5.1 | 0.0 | 1.6 | 0.6 | 0.4 | 0.4 | 32 | 3.4 | 0.0 | 1.5 | 0.6 | 0.3 | 0.4 |
| 5 | 15.4 | 3.4 | 1.7 | 0.3 | 0.1 | 0.3 | 24 | 13.7 | 1.7 | 1.6 | 0.4 | 0.1 | 0.2 |
| 6 | 17.1 | 3.8 | 0.8 | 0.4 | 0.2 | 0.3 | 26 | 15.4 | 3.4 | 0.6 | 0.4 | 0.3 | 0.4 |
| 7 | 12.0 | 3.4 | 1.0 | 0.4 | 0.3 | 0.4 | 21 | 13.7 | 5.1 | 1.2 | 0.4 | 0.2 | 0.3 |
| 8 | 13.7 | 1.7 | 1.7 | 0.7 | 0.4 | 0.4 | 32 | 6.8 | 0.0 | 1.6 | 0.6 | 0.3 | 0.4 |
| 9 | 17.1 | 4.1 | 1.4 | 0.4 | 0.1 | 0.3 | 27 | 15.4 | 3.4 | 1.4 | 0.4 | 0.2 | 0.4 |
| 10 | 12.0 | 0.0 | 1.3 | 0.5 | 0.2 | 0.3 | 28 | 6.8 | 0.0 | 1.4 | 0.5 | 0.2 | 0.3 |
| 11 | 15.4 | 3.4 | 1.6 | 0.7 | 0.4 | 0.5 | 22 | 12.0 | 1.7 | 1.4 | 0.6 | 0.3 | 0.5 |
| 12 | 12.0 | 0.0 | 1.4 | 0.5 | 0.2 | 0.3 | 26 | 5.1 | 0.0 | 1.4 | 0.6 | 0.2 | 0.3 |
| 13 | 3.4 | 0.0 | 1.3 | 0.4 | 0.1 | 0.2 | 21 | 5.1 | 1.7 | 0.9 | 0.4 | 0.1 | 0.2 |
| 14 | 10.3 | 3.1 | 1.4 | 0.5 | 0.3 | 0.4 | 24 | 8.6 | 1.7 | 1.3 | 0.6 | 0.2 | 0.3 |
| 15 | 13.7 | 1.7 | 1.7 | 0.7 | 0.3 | 0.5 | 29 | 6.8 | 1.7 | 1.7 | 0.6 | 0.3 | 0.4 |
| 16 | 10.3 | 3.4 | 1.4 | 0.4 | 0.1 | 0.2 | 22 | 8.6 | 1.7 | 1.4 | 0.4 | 0.2 | 0.3 |

Abbreviations: TB = total bilirubin (µmol/L); CB = conjugated bilirubin (µmol/L); AP = alkaline phosphatase (µkat/L); GGT = gamma-glutamyltranspeptidase (µkat/L); AST = aminotransferase aspartate (µkat/L); ALT = aminotransferase alanine (µkat/L).

In group B, to which only TPN was administered, 10 patients in 21 (47%) developed intrahepatic cholestasis, characterised by an increase in total and conjugate bilirubin, alkaline phosphatase, $\gamma$-GT and transaminase after 13-22 days of treatment (average 17.5 days) as shown in Table III. None of these patients received simultaneous treatment with other drugs. When cholestasis arose, these 10 patients were treated intravenously with SAMe-1,4-butanedisulphonate at a daily dose of 800 mg of active substance in association with the parenteral nutrition treatment scheme underway. After the first week of treatment with SAMe a statistically significant improvement ($p < 0.01$) was observed in all biochemical parameters, as shown in Tables III and IV. On termination of the trial period (suspension of total parenteral nutrition; average duration of treatment with SAMe 12.3 days), all the considered biochemical parameters were completely normal (Tables III and IV).

TABLE III- Group B: Biochemical parameter values at beginning of trial, on appearance of cholestasis (average 17.5 days), after the first week and on termination of treatment with SAMe-1,4-butanedisulphonate (800 mg/day i.v.) in association with total parenteral nutrition (TPN) (average 12.3 days) on the 10 patients of group B who developed cholestasis.

EP 0 501 293 A1

| Pat. | Beginning of trial | | | | | | Appearance of cholestasis | | | | | | | |
|------|------|------|------|------|------|------|------|------|------|------|------|------|------|-------------|
| No. | TB | CB | AP | GGT | AST | ALT | Day | TB | CB | AP | GGT | AST | ALT | SAMe + TPN |
| 2 | 13.7 | 3.1 | 1.6 | 0.3 | 0.3 | 0.3 | 19 | 53.1 | 27.4 | 3.7 | 0.8 | 1.2 | 1.7 | 800 mg/day |
| 3 | 12.0 | 3.4 | 1.5 | 0.5 | 0.4 | 0.5 | 13 | 73.5 | 47.9 | 5.8 | 1.3 | 1.4 | 2.4 | 800 mg/day |
| 6 | 8.6 | 3.4 | 1.4 | 0.3 | 0.1 | 0.2 | 21 | 44.5 | 15.4 | 4.8 | 1.2 | 1.2 | 2.2 | 800 mg/day |
| 7 | 15.4 | 4.1 | 1.7 | 0.4 | 0.2 | 0.4 | 16 | 68.4 | 49.6 | 6.2 | 1.2 | 1.4 | 2.5 | 800 mg/day |
| 9 | 8.6 | 3.4 | 1.7 | 0.4 | 0.1 | 0.2 | 18 | 39.3 | 23.9 | 4.9 | 0.8 | 0.2 | 0.4 | 800 mg/day |
| 11 | 15.4 | 3.4 | 1.9 | 0.7 | 0.2 | 0.3 | 17 | 53.1 | 32.5 | 4.7 | 0.7 | 1.4 | 2.2 | 800 mg/day |
| 15 | 12.0 | 3.4 | 1.6 | 0.4 | 0.1 | 0.2 | 14 | 49.6 | 22.2 | 4.7 | 0.5 | 1.3 | 2.4 | 800 mg/day |
| 18 | 5.1 | 1.7 | 1.4 | 0.6 | 0.3 | 0.4 | 18 | 7.1 | 49.6 | 6.4 | 1.3 | 1.7 | 3.3 | 800 mg/day |
| 20 | 8.6 | 1.7 | 1.3 | 0.6 | 0.3 | 0.4 | 22 | 66.7 | 39.3 | 6.2 | 0.9 | 1.5 | 3.0 | 800 mg/day |
| 21 | 5.1 | 0.0 | 1.1 | 0.5 | 0.3 | 0.4 | 17 | 73.5 | 53.1 | 5.7 | 0.9 | 1.5 | 3.2 | 800 mg/day |

TABLE III (continued)

| Pat. | First week (SAMe + TPN) | | | | | | | End of trial (SAMe + TPN) | | | | | |
|------|------|------|------|------|------|------|------|------|------|------|------|------|------|
| No. | TB | CB | AP | GGT | AST | ALT | Day | TB | CB | AP | GGT | AST | ALT |
| 2 | 27.4 | 8.6 | 1.9 | 0.7 | 0.9 | 1.3 | 31 | 15.4 | 1.7 | 2.0 | 0.6 | 0.4 | 0.4 |
| 3 | 18.8 | 1.3 | 2.5 | 0.9 | 0.7 | 0.8 | 25 | 17.1 | 3.8 | 2.0 | 0.6 | 0.5 | 0.6 |
| 6 | 17.1 | 6.8 | 2.7 | 0.6 | 0.7 | 0.7 | 34 | 13.7 | 1.7 | 1.5 | 0.6 | 0.3 | 0.4 |
| 7 | 30.8 | 15.4 | 2.8 | 0.9 | 0.8 | 0.9 | 29 | 12.0 | 3.4 | 1.5 | 0.7 | 0.4 | 0.5 |
| 9 | 15.4 | 5.1 | 1.5 | 0.7 | 0.3 | 0.3 | 27 | 13.7 | 3.1 | 1.5 | 0.7 | 0.2 | 0.3 |
| 11 | 13.7 | 1.7 | 2.2 | 0.6 | 0.7 | 0.8 | 31 | 12.0 | 1.7 | 1.9 | 0.6 | 0.4 | 0.5 |
| 15 | 12.0 | 3.4 | 1.7 | 0.5 | 0.8 | 0.9 | 29 | 10.3 | 1.7 | 1.6 | 0.5 | 0.4 | 0.5 |
| 18 | 15.4 | 5.1 | 1.7 | 0.7 | 0.9 | 1.2 | 30 | 8.6 | 3.4 | 1.6 | 0.6 | 0.2 | 0.4 |
| 20 | 17.1 | 6.8 | 2.2 | 0.7 | 0.8 | 0.9 | 34 | 5.1 | 1.7 | 1.2 | 0.5 | 0.3 | 0.4 |
| 21 | 13.7 | 3.4 | 1.7 | 0.6 | 0.5 | 0.7 | 28 | 6.8 | 1.7 | 1.1 | 0.5 | 0.4 | 0.4 |

Abbreviations: TB = total bilirubin ($\mu$mol/L); CB = conjugated bilirubin ($\mu$mol/L); AP = alkaline phosphatase ($\mu$kat/L); GGT = gamma-glutamyltranspeptidase ($\mu$kat/L); AST = aminotransferase aspartate ($\mu$kat/L); ALT = aminotransferase alanine ($\mu$kat/L).

TABLE IV - Group B: Biochemical parameters (mean ± SE) at the beginning, after one week and on termination of treatment with SAMe-1,4-butanedisulphonate (800 mg/day i.v.; average 12.3 days) in association with total parenteral nutrition in the 10 patients of group B who developed cholestasis.

| | Beginning | One week | Therapy termination |
|---|---|---|---|
| TB (μmol/L) | 59.2 ± 3.9 | 18.1 ± 1.9* | 11.5 ± 1.2* |
| CB (μmol/L) | 36.1 ± 4.3 | 6.7 ± 1.2* | 2.4 ± 0.3* |
| AP (μkat/L) | 5.3 ± 0.3 | 1.9 ± 0.1* | 1.6 ± 0.1* |
| GGT (μkat/L) | 0.9 ± 0.1 | 0.7 ± 0.0* | 0.6 ± 0.0* |
| AST (μkat/L) | 1.3 ± 0.1 | 0.7 ± 0.1* | 0.3 ± 0.0* |
| ALT (μkat/L) | 2.2 ± 0.2 | 0.8 ± 0.1* | 0.4 ± 0.0* |

* $p < 0.01$ against initial value (ANOVA BR)

Abbreviations: TB = total bilirubin; CB = conjugated bilirubin; AP = alkaline phosphatase; GGT = gamma-glutamyltranspeptidase; AST = aminotransferase aspartate; ALT = aminotransferase alanine.

None of the patients treated with SAMe (16 patients of group A; 10 patients of group B) complained of side effects deriving from the treatment underway. Hence in no case was it necessary to suspend the therapy or reduce the SAMe dosage.

Table V shows the values of the biochemical parameters of those patients of group B who did not develop cholestasis.

TABLE V - Values of the biochemical parameters at the beginning and end of the trial (average 32 days) in the 11 patients of group B who did not develop cholestasis.

| Pat. No. | Beginning of trial | | | | | | Day | End of trial | | | | | |
|---|---|---|---|---|---|---|---|---|---|---|---|---|---|
| | TB | CB | AP | GGT | AST | ALT | | TB | CB | AP | GGT | AST | ALT |
| 1 | 10.3 | 1.7 | 1.9 | 0.5 | 0.4 | 0.4 | 30 | 12.0 | 1.7 | 1.7 | 0.6 | 0.3 | 0.5 |
| 4 | 17.1 | 3.4 | 1.9 | 0.7 | 0.2 | 0.3 | 28 | 13.7 | 3.4 | 1.5 | 0.5 | 0.3 | 0.5 |
| 5 | 6.8 | 1.7 | 1.1 | 0.3 | 0.2 | 0.4 | 26 | 8.6 | 1.7 | 1.2 | 0.4 | 0.2 | 0.3 |
| 8 | 6.8 | 1.7 | 1.6 | 0.6 | 0.3 | 0.4 | 31 | 8.6 | 1.7 | 1.5 | 0.7 | 0.3 | 0.5 |
| 10 | 12.0 | 2.1 | 1.5 | 0.5 | 0.3 | 0.4 | 42 | 10.3 | 3.4 | 1.6 | 0.5 | 0.1 | 0.4 |
| 12 | 12.0 | 1.7 | 1.4 | 0.6 | 0.3 | 0.4 | 37 | 6.8 | 1.7 | 1.3 | 0.7 | 0.2 | 0.5 |
| 13 | 18.8 | 1.7 | 1.3 | 0.4 | 0.3 | 0.4 | 24 | 17.1 | 3.4 | 1.3 | 0.5 | 0.2 | 0.4 |
| 14 | 13.7 | 3.1 | 1.9 | 0.8 | 0.3 | 0.4 | 30 | 12.0 | 1.7 | 1.5 | 0.5 | 0.3 | 0.5 |
| 16 | 15.4 | 4.1 | 1.4 | 0.5 | 0.2 | 0.3 | 39 | 10.3 | 1.7 | 1.2 | 0.3 | 0.3 | 0.4 |
| 17 | 6.8 | 1.7 | 1.5 | 0.5 | 0.3 | 0.4 | 45 | 6.8 | 1.7 | 1.4 | 0.5 | 0.3 | 0.5 |
| 19 | 1.7 | 0.0 | 1.2 | 0.4 | 0.1 | 0.2 | 21 | 6.8 | 1.7 | 1.4 | 0.5 | 0.3 | 0.4 |

Abbreviations: TB = total bilirubin ($\mu$mol/L); CB = conjugated bilirubin ($\mu$mol/L); AP = alkaline phosphatase ($\mu$kat/L); GGT = gamma-glutamyltranspeptidase ($\mu$kat/L); AST = aminotransferase aspartate ($\mu$kat/L); ALT = aminotransferase alanine ($\mu$kat/L).

As can be seen from the aforegoing tables, intravenous treatment with SAMe at a daily dose of 800 mg is effective in preventing the onset of intrahepatic cholestasis in patients subjected to total parenteral nutrition. Cholestasis is in fact observed only in 1 patient in 16 (6%) of group A, whereas 10 patients in 21 (47%) of group B showed an increase in the biochemical parameters relating to cholestasis.

In addition, treatment with SAMe proved effective in resolving cholestasis in all 10 patients of group B who had developed intrahepatic cholestasis. In all these cases an improvement in biochemical parameters was already observed after the first week of treatment, the parameters returning to normal by the end of the trial.

Tolerance under SAMe treatment was good for all patients. No noticeable side effects were recorded.

The following examples of injectable pharmaceutical compositions containing pharmaceutically acceptable salts of SAMe (200, 300, 400 and 500 mg of active substance) for the prevention and symptomatic treatment of intrahepatic cholestasis consequent on total parenteral nutrition are given for the purposes of illustration. The preferred SAMe salts are the disulphate-p-toluenesulphonate, the 1,4-butanedisulphonate and the 2,5-sulphate.

The injectable compositions are prepared as follows:

the content of a vial consisting of water containing L-lysine (160-640 mg) and sodium hydroxide (2,18 mg) are added to a bottle containing the SAMe salt possibly mixed with mannitol.

EXAMPLE 1

- a bottle contains:

SAMe disulphate-p-toluenesulphonate     384 mg

(equivalent to 200 mg of active substance)

Mannitol     240 mg

- a solvent vial contains:

L-lysine     300 mg

Sodium hydroxide     9 mg

$H_2O$ for injectable solutions   to make up to   5 ml

EXAMPLE 2

- a bottle contains:

SAMe 1,4-butanedisulphonate     394 mg

(equivalent to 200 mg of active substance)

- a solvent vial contains:

L-lysine     160 mg

Sodium hydroxide     4.5 mg

$H_2O$ for injectable solutions   to make up to   2.5 ml

EXAMPLE 3

- a bottle contains:

SAMe 2,5-sulphate     333 mg

(equivalent to 200 mg of active substance)

- a solvent vial contains:

L-lysine     320 mg

Sodium hydroxide     2 mg

$H_2O$ for injectable solutions   to make up to   5 ml

EXAMPLE 4

- a bottle contains:

SAMe disulphate-p-toluenesulphonate     768 mg

(equivalent to 400 mg of active substance)

- a solvent vial contains:

L-lysine     600 mg

Sodium hydroxide     18 mg

$H_2O$ for injectable solutions   to make up to 10 ml

EXAMPLE 5

- a bottle contains:

SAMe 1,4-butanedisulphonate     788 mg

(equivalent to 400 mg of active substance)

- a solvent vial contains:

L-lysine     305 mg

Sodium hydroxide     2.1 mg

$H_2O$ for injectable solutions   to make up to 5 ml

EXAMPLE 6

- a bottle contains:

SAMe 2,5-sulphate     670 mg

(equivalent to 400 mg of active substance)

- a solvent vial contains:

L-lysine     640 mg

Sodium hydroxide     4 mg

$H_2O$ for injectable solutions   to make up to 5 ml

EXAMPLE 7

- a bottle contains:

SAMe 1,4-butanedisulphonate        591 mg

(equivalent to 300 mg of active substance)

- a solvent vial contains:

L-lysine        218 mg

Sodium hydroxide        7.4 mg

$H_2O$ for injectable solutions    to make up to   5 ml

EXAMPLE 8

- a bottle contains:

SAMe 1,4-butanedisulphonate        985 mg

(equivalent to 500 mg of active substance)

- a solvent vial contains:

L-lysine        351 mg

Sodium hydroxide        11.7 mg

$H_2O$ for injectable solutions    to make up to   5 ml

## Claims

1. The use of S-adenosyl-L-methionine (SAMe) in the form of pharmaceutically acceptable salts for the preparation of injectable pharmaceutical compositions for the prevention and symptomatic treatment of intrahepatic cholestasis determined by total parenteral nutrition.

2. The use claimed in claim 1, characterised in that said pharmaceutically acceptable salts are the disulphate-p-toluenesulphonate, the 1,4-butanedisulphonate or the 2,5-sulphate salt.

3. The use claimed in claim 1, characterised in that said composition is in the form of a bottle containing the SAMe salt in a quantity of between 200 and 500 mg of active substance, and a vial of solvent consisting of between 2.5 and 10 ml of $H_2O$ containing between 160 and 640 mg of L-lysine and between 2 and 18 mg of sodium hydroxide.

**Claims for the following Contracting States : GR, ES**

1. A process for preparing injectable pharmaceutical composition for the prevention and sympomatic treatment of intrahepatic cholestasis determined by total parenteral nutrition and comprising as the active principle S-adenosyl-L-methionine (SAMe) or at least one of its pharmacologically acceptable salt, said process consisting of mixing said active principle with suitable diluents and excipients.

2. The process according to claim 1, wherein said pharmaceutically acceptable salts are the disulphate-p-toluenesulphonate, the 1,4-butanedisulphonate and the 2,5-sulphate.

3. The process according to claim 1, wherein a vial consisting of from 2.5 to 10 ml of water containing between 160 and 640 mg of L-lysine and between 2 and 18 mg of sodium hydroxide is added to a bottle containing a quantity of between 200 and 500 mg of SAMe salt.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | DRUGS, vol. 40, suppl. 3, 1990, pages 129-138, Adis International Ltd; "Round-table discussion" * Pages 130,131 * | 1,2 | A 61 K 31/70 |
| Y | IDEM | 3 | |
| X | DRUGS, vol. 40, suppl. 3, 1990, pages 111-123, Adis International Ltd; P. ALMASIO et al.: "Role of S-adenosyl-L-methionine in the treatment of intrahepatic cholestasis" * Whole article * | 1,2 | |
| Y | IDEM | 3 | |
| X | METH. FIND. EXP. CLIN. PHARMACOL., vol. 12, no. 1, 1990, pages 69-78, J.R. Prous, S.A.; M. COLTORTI et al.: "A review of the studies on the clinical use of S-adenosylmethionine (SAMe) for the symptomatic treatment of intrahepatic cholestasis" * Whole article * | 1,2 | TECHNICAL FIELDS SEARCHED (Int. Cl.5) A 61 K |
| Y | IDEM | 3 | |
| Y | EP-A-0 136 463 (BIORESEARCH S.p.A.) * Whole document * | 3 | |
| Y | EP-A-0 387 756 (BIORESEARCH S.p.A.) * Page 6, example 8; page 7, examples 9,10; claims 1,3,9,11 * | 3 | |

The present search report has been drawn up for all claims

| Place of search | Date of completion of the search | Examiner |
|---|---|---|
| THE HAGUE | 29-05-1992 | ORVIZ DIAZ P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document